# EUROPEAN PATENT APPLICATION

(11) **EP 4 765 082 A1**
(43) Date of publication of application: **24.06.2026**
(21) Application number: 24383447.0
(22) Date of filing: 23.12.2024
(51) Int. Cl.: G09F 27/00, H05K 5/00, H05K 5/02, H05K 5/06, H05K 7/20

(54) **AN AUDIO-VISUAL EQUIPMENT FOR AN OPERATING ROOM**

(71) Applicant: Albiral Display Solutions, S.L., 08512 Sant Hipòlit De Voltregà, Barcelona (ES)
(72) Inventor: MANAUT COSTA, Miquel dels Sants, 08512 SANT HIPÒLIT DE VOLTREGÀ (ES)
(74) Representative: Ponti & Partners, S.L.P

(57) **Abstract**

The present invention relates to an audio-visual equipment for an operating room, comprising a cabinet comprising:
- an enclosure (2) for housing electrical and electronic components, the enclosure (2) having a front opening (2a), and
- a front door (1) hinged to the enclosure (2) to, when in a closed position, close the front opening (2a) in a water- and dust-proof sealing manner.

The audio-visual equipment further comprises a ventilation system comprising vent holes (V1, V2) defined in a back face and/or side face of the enclosure (2) and is configured and arranged for cooling the electrical and electronic components by air convection through said vent holes (V1, V2), without forced ventilation.

## Description

### FIELD OF THE INVENTION

The present invention relates to an audio-visual equipment for an operating room, which provides high performance in terms of avoiding contamination of the operating room.

### BACKGROUND OF THE INVENTION

Audio-visual equipment for operating rooms comprising the features of the preamble of claim 1 is known in the art, i.e. comprising a cabinet comprising the following features:
- an enclosure for housing electrical and electronic components, said enclosure having a front opening, and
- a front door hinged to the enclosure to, when in a closed position, close said front opening in a water- and dust-proof sealing manner.

Some of said known concealable apparatus assemblies are disclosed in CN212933495U.

Those already known audio-visual equipment have some shortcomings, such as the generation of air flows carrying dust which can enter the operating room, and therefore contaminate the air inside the operating room, thus not meeting the requested standards (such as the ISO 4406 standard) and/or requesting a higher frequency in the required air changes per hour (ACH).

It is, therefore, necessary to provide an alternative to the state of the art which covers the gaps found therein, by providing an audio-visual equipment for an operating room which overcomes the above mentioned shortcomings.

### SUMMARY OF THE INVENTION

To that end, the present invention relates to an audio-visual equipment for an operating room, comprising a cabinet comprising:
- an enclosure for housing electrical and electronic components, said enclosure having a front opening, and
- a front door hinged to said enclosure to, when in a closed position, close said front opening in a water- and dust-proof sealing manner, preferably providing at least an IP65 rating protection.

In contrast to the audio-visual equipment for operating rooms known in the prior art, in the one of the present invention, in a characterizing manner, the audio-visual equipment further comprises a ventilation system comprising vent holes defined in a back face and/or side face of the enclosure and is configured and arranged for cooling said electrical and electronic components by air convection through said vent holes, without forced ventilation. Therefore, no air flow carrying dust which can enter the operating room is forcedly generated.

According to an embodiment, the front door comprises an inner perimeter magnetic sealing gasket to magnetically attach to respective magnetically attractable areas of the enclosure surrounding the front opening.

Generally, the enclosure has a parallelepipedal shape, with a frame of four side walls and a back wall attached thereto.

For a preferred embodiment, the enclosure is embeddable into a wall of the operating room, although for other, less preferred, embodiments the enclosure is wall-mountable. In both cases, access to its interior (for example, for maintaining purposes) is achieved by opening the front door.

Those vent holes are defined at parts of the enclosure not exposed to the inside of the operating room.

Therefore, for the embeddable enclosure embodiment, the vent holes are defined at the back wall of the enclosure and/or at side walls thereof, as all of those wall remain not exposed to the inside of the operating room, while for the wall-mountable enclosure, the vent holes are defined at the back wall, as only that back wall remains not exposed to the inside of the operating room.

By means of those vent holes arrangements/locations, not even the air flow cause by natural convection withing the cabinet is delivered to the inside of the operating room, thus even further increasing the lack of contamination thereof, meeting the requested standards (such as the ISO 4406 standard) and requesting a lower frequency in the required air changes per hour (ACH).

For an embodiment, the front door is hinged to the enclosure to, in use, pivot about a vertical rotation axis.

For an embodiment, the cabinet further comprises a front metallic plate and the audio-visual equipment further comprises an excitor speaker housed within the enclosure and attached to a back face of the front metallic plate to make it vibrate to transmit sound, the front metallic plate being attached to the enclosure in a water- and dust-proof sealing manner. By means of that excitor speaker, in contrast to prior art equipment that uses other types of speakers, no holes for transmitting sound are needed, so that the risk of contaminating the inside of the operating room is even further decreased.

According to an implementation of that embodiment, the audio-visual equipment further comprises at least one push-button and/or at least one connector, operatively connected to the electrical and electronic components and tightly inserted into respective through holes of the metallic plate in a sealed manner, so that no water or dust can penetrate there through.

For an embodiment, the audio-visual equipment further comprising a cap for covering the end of the at least one connector that is accessible from outside the cabinet, so that no water can penetrate there through, for example when cleaning the equipment with water.

According to an embodiment, the audio-visual equipment further comprises a water- and dust-proof keyboard assembly attached to the front door through a water-and dust-proof perimeter seal.

For an embodiment, the electrical and electronic components include a display and a camera, and respective transparent regions of the front door are coincident, when the front door is closed, with that display and camera, so that what is displayed in the display can be seen from outside the equipment and the camera can acquire images of objects, persons or scenes located outside the equipment.

According to an embodiment, the electrical and electronic components comprise a chronometer, and wherein a transparent region of the front door is coincident, when the front door is closed, with a display area of that chronometer, so that what is displayed in that display area can be seen from outside the equipment.

For an embodiment, the electrical and electronic components comprise a water-and dust-proof microphone at least partially tightly inserted into a through hole of the front door in a sealed manner, so that no water or dust can penetrate there through.

Preferably, all the water- and dust-proof sealings mentioned above, for different embodiments, provide at least an IP65 rating protection.

### BRIEF DESCRIPTION OF THE FIGURES

In the following some preferred embodiments of the invention will be described with reference to the enclosed figures, particularly embodiments for which the bendable elongate apparatus is a bendable elongate microphone. They are provided only for illustration purposes without however limiting the scope of the invention.
Figure 1 is a perspective front view that shows the audio-visual equipment of the present invention, for an embodiment, where the front door of the cabinet is in a closed position.
Figure 2 shows a side elevational view (left) and a front elevational view (right) of the audio-visual equipment of the present invention, for the same embodiment as Figure 1.
Figure 3 shows a perspective front view analogous to that of Figure 1, but where the front door of the cabinet is in an open position, showing the components housed within the enclosure.
Figure 4 is a front elevational view analogous to that of Figure 3, but where the front door of the cabinet is in a partially open position, showing the components housed within the enclosure and also those attached to the back face of the front door.
Figure 5 shows at its right view a front elevational view of the enclosure of the cabinet of the audio-visual equipment of the present invention, without the front door, and at its left view an elevational cross-section taken along the cut plane represented by line C-C in the right view.
Figure 6 is an enlarged view of the elements arranged within the dashed line circle in the left view of Figure 5.

### DESCRIPTION OF THE PREFERRED EMBODIMENTS

In the present section an embodiment of the concealable apparatus assembly of the present invention will be described with reference to the Figures. In the following (and also in the previous sections), the use of positional terms, such as back, upper, lower, front, side, etc., in the present document correspond to the position illustrated in the enclosed figures.

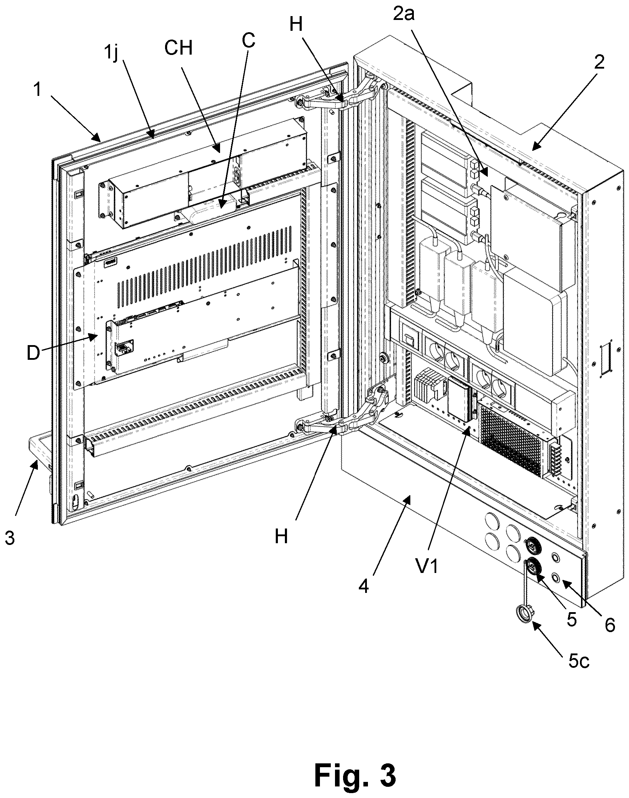

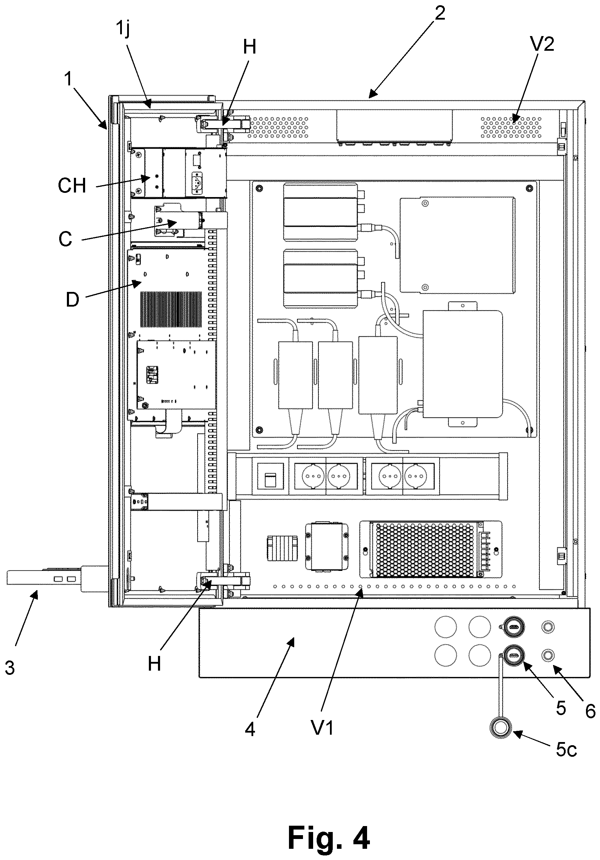

As shown in the Figures, the present invention relates to an audio-visual equipment for an operating room, comprising a cabinet comprising:
- an enclosure 2 for housing electrical and electronic components, the enclosure 2 having a parallelepipedal shape, with a frame of four side walls and a back wall attached thereto, and a front opening 2a.
- a front door 1 hinged to the enclosure 2 to, when in a closed position, close the front opening 2a in a water- and dust-proof sealing manner, particularly, as shown in Figures 3 and 4, by means of an inner perimeter magnetic sealing gasket 1j to magnetically attach to respective magnetically attractable areas of the enclosure 2 surrounding the front opening 2a. In addition, the front door 1 is preferably closed by means of two clips to prevent opening.

Depending on the embodiment, the enclosure 2 is embeddable into a wall or wall-mountable, such that access to its interior is achieved by opening the front door 1.

As shown especially in Figures 3, 4 and 5, the audio-visual equipment further comprises a ventilation system comprises vent holes V1, V2 defined in a back face of the enclosure 2 and is configured and arranged for cooling said electrical and electronic components by air convection through said vent holes V1, V2, without forced ventilation.

Particularly, a lower group of vent holes V1 and an upper group of vent holes V2 are arranged in that back face of the enclosure 2.

The lower group of vent holes V1 are configured and arranged to allow the entry of fresh air into the cabinet, while the upper group of vent holes V2 are configured and arranged to allow hot air to exit the cabinet.

As shown in Figures 3 and 4, the front door 1 is hinged to the enclosure 2, particularly by means of two hinges H, to, in use, pivot about a vertical rotation axis.

For the illustrated embodiment, the electrical and electronic components include, among others, a display D, a camera C, and a chronometer CH, as shown in Figures 3 and 4, which are particularly attached to a back face of the front door 1.

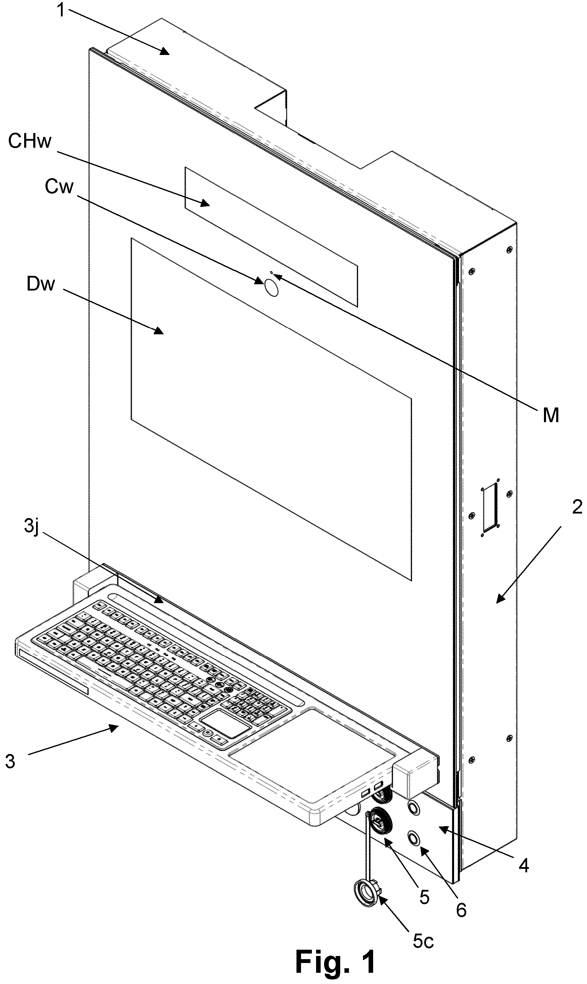

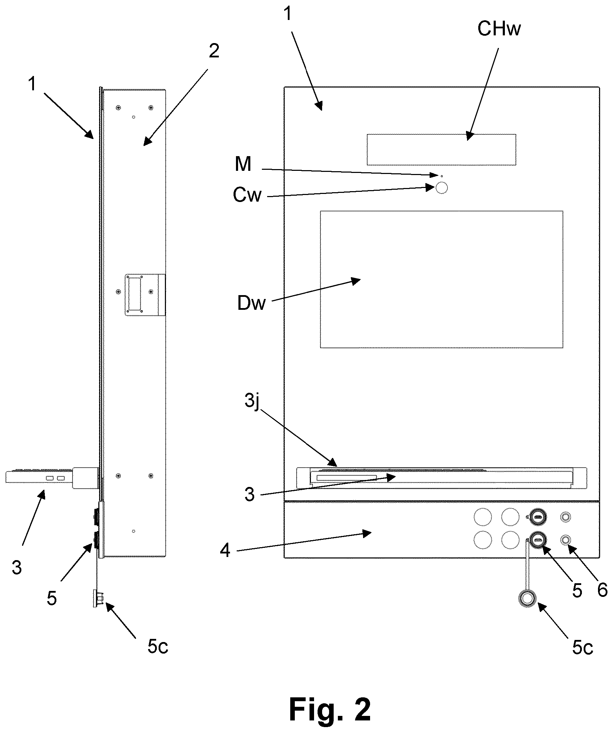

As shown in Figures 1 and 2 (right view), the front door 1 includes respective transparent regions Dw, Cw, CHw coincident, when the front door 1 is closed, with the display D, camera C, and chronometer.

In addition, for the illustrated embodiment, as shown in Figures 1 and 2 (right view), the electrical and electronic components further comprises a water-and dust-proof microphone M at least partially tightly inserted into a through hole of the front door 1 in a sealed manner, so that no water or dust can penetrate there through.

As shown in Figures 1-4, the audio-visual equipment further comprises a water-and dust-proof keyboard assembly 3 attached to the front door 1 through a water- and dust-proof perimeter sealing gasket 3j. in this case, the water- and dust-proof keyboard assembly 3 includes a keyboards and an adjacent keypad, although other types of keyboard assemblies can be similarly arranged, for other embodiments, and/or other types of input devices, such as a mouse.

As shown in Figures 1, 2, 4, 5 and 6, the cabinet further comprises a front metallic plate 4 (for example, made of aluminium) being attached to the enclosure 2 in a water- and dust-proof sealing manner, by means of a perimeter sealing gasket 4j. In the illustrated embodiment, the front metallic plate 4 is arranged below the front opening 2a and is flushed with the front door 1, when the latter is closed. However, for other embodiments (not illustrated), the location of the front metallic plate 4 may be different.

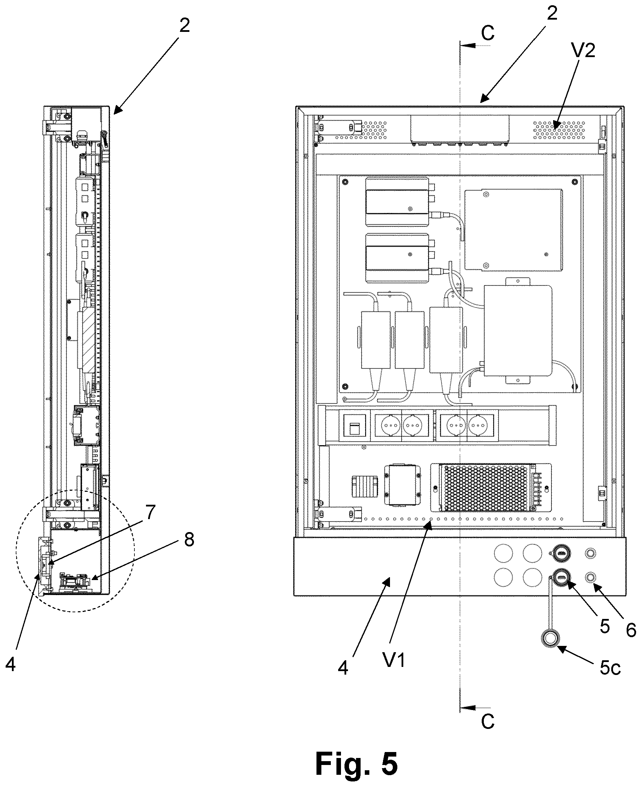

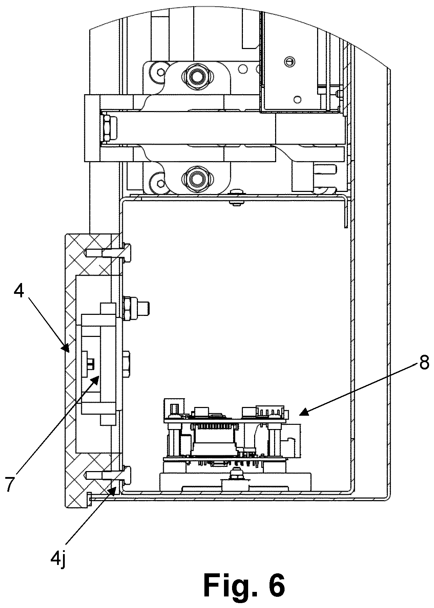

As shown in Figure 5, left view, and especially in Figure 6, the audio-visual equipment further comprises one or more excitor speakers 7 housed within the enclosure 2 and attached to a back face of the front metallic plate 4 to make it vibrate to transmit sound. An audio controller 8 is also provided, housed within the enclosure 2, and electrically and operatively connected to the speaker(s) 7, by means of corresponding wiring (not shown, for clarity's sake).

In addition, as shown in Figures 1-5, the audio-visual equipment further comprising two push-buttons 6 and two connectors 5, although the number of push-buttons 6 and connectors 5 may be different to two (for non-illustrated embodiments).

The push-buttons 6 and connectors 5 are electrically and operatively connected to the electrical and electronic components and tightly inserted into respective through holes of the metallic plate 4 in a sealed manner, so that no water or dust can penetrate there through.

When assembling each connector 5, sealing is ensured by placing a sealing gasket (not shown) between the connector 5 and the metallic plate 4. When tightening with the connector with a nut, the sealing is ensured.

As shown also in Figures 1-5, the audio-visual equipment further comprises a cap 5c for covering the end of the connector 5 that is accessible from outside the cabinet, so that no water can penetrate there through, for example when cleaning the equipment with water. Although only one cap 5c has been illustrated, preferably there is a cap 5c for each connector 5.

Further electric and electronic components are included in the audio-visual equipment of the present invention, which remain at least partially housed withing the enclosure 2 when the front door 1 is close. Some of those components are shown in the Figure , such as data processors, radiators, etc., while others have not been depicted, for clarity's sake, such as wiring arrangements for electrically and operatively connect the different components with corresponding power supplies, data processors, I/O boards, sound boards, graphic boards, etc.

A person skilled in the art could introduce changes and modifications in the embodiments described without departing from the scope of the invention as it is defined in the attached claims.

## Claims

1. An audio-visual equipment for an operating room, comprising a cabinet comprising:
- an enclosure (2) for housing electrical and electronic components, said enclosure (2) having a front opening (2a), and
- a front door (1) hinged to said enclosure (2) to, when in a closed position, close said front opening (2a) in a water- and dust-proof sealing manner,
**characterized in that** the audio-visual equipment further comprises a ventilation system comprising vent holes (V1, V2) defined in a back face and/or side face of the enclosure (2) and is configured and arranged for cooling said electrical and electronic components by air convection through said vent holes (V1, V2), without forced ventilation.

2. The audio-visual equipment according to claim 1, wherein said front door (1) is hinged to said enclosure (2) to, in use, pivot about a vertical rotation axis.

3. The audio-visual equipment according to any of the previous claims, wherein said cabinet further comprises a front metallic plate (4) and audio-visual equipment further comprises an excitor speaker (7) housed within said enclosure (2) and attached to a back face of said front metallic plate (4) to make it vibrate to transmit sound, the front metallic plate (4) being attached to the enclosure (2) in a water- and dust-proof sealing manner.

4. The audio-visual equipment according to claim 3, further comprising at least one push-button (6) and/or at least one connector (5), operatively connected to said electrical and electronic components and tightly inserted into respective through holes of the metallic plate (4) in a sealed manner, so that no water or dust can penetrate there through.

5. The audio-visual equipment according to claim 4, further comprising a cap (5c) for covering the end of said at least one connector (5) that is accessible from outside the cabinet, so that no water can penetrate there through.

6. The audio-visual equipment according to any of the previous claims, wherein said front door (1) comprises an inner perimeter magnetic sealing gasket (1j) to magnetically attach to respective magnetically attractable areas of the enclosure (2) surrounding the front opening (2a).

7. The audio-visual equipment according to any of the previous claims, wherein said water- and dust-proof sealing manner provides at least an IP65 rating protection.

8. The audio-visual equipment according to any of the previous claims, further comprising a water- and dust-proof keyboard assembly (3) attached to said front door (1) through a water- and dust-proof perimeter sealing gasket (3j).

9. The audio-visual equipment according to any of the previous claims, wherein said electrical and electronic components include a display (D) and a camera (C), and wherein respective transparent regions (Dw, Cw) of said front door (1) are coincident, when the front door (1) is closed, with said display (D) and said camera (C).

10. The audio-visual equipment according to any of the previous claims, wherein the electrical and electronic components comprise a chronometer (CH), and wherein a transparent region (CHw) of the front door (1) is coincident, when the front door (1) is closed, with a display area of said chronometer (CH).

11. The audio-visual equipment according to any of the previous claims, wherein the electrical and electronic components comprise a water-and dust-proof microphone (M) at least partially tightly inserted into a through hole of the front door (1) in a sealed manner, so that no water or dust can penetrate there through.

12. The audio-visual equipment according to any of the previous claims, wherein the enclosure (2) is embeddable into a wall, such that access to its interior is achieved by opening the front door (1).
